Europäisches Patentamt

European Patent Office

Office européen des brevets

(11) Numéro de publication : **0 419 367 B1**

(12) **FASCICULE DE BREVET EUROPEEN**

(45) Date de publication du fascicule du brevet :
**08.06.94 Bulletin 94/23**

(51) Int. Cl.$^5$ : **G01N 33/53,** G01N 33/577,
G01N 33/68, G01N 33/543,
G01N 33/74, G01N 33/569,
C12Q 1/68

(21) Numéro de dépôt : **90402611.9**

(22) Date de dépôt : **20.09.90**

(54) **Procédé de détection d'une substance biologique à l'aide de ligands.**

(30) Priorité : **20.09.89 FR 8912354**

(43) Date de publication de la demande :
**27.03.91 Bulletin 91/13**

(45) Mention de la délivrance du brevet :
**08.06.94 Bulletin 94/23**

(84) Etats contractants désignés :
**BE CH DE ES FR GB IT LI NL SE**

(56) Documents cités :
**WO-A-85/04422**
**GB-A- 2 161 165**
**GB-A- 2 171 999**
**GB-A- 2 189 810**

(73) Titulaire : **BIO MERIEUX, Société anonyme**
**F-69280 Marcy l'Etoile (FR)**

(72) Inventeur : **Mandrand, Bernard**
**21, petite rue de la Doua**
**F-69100 Villeurbanne (FR)**
Inventeur : **Archinard, Philippe**
**11, avenue Félix Faure**
**F-69007 Lyon (FR)**
Inventeur : **Charles, Marie-Hélène**
**Chemin du Vernon**
**F-69420 Condrieu (FR)**
Inventeur : **Trouyez, Gérard**
**30, rue Chazière**
**F-69004 Lyon (FR)**

(74) Mandataire : **Tonnellier, Jean-Claude et al**
**Cabinet Nony & Cie.**
**29, rue Cambacérès**
**F-75008 Paris (FR)**

EP 0 419 367 B1

## Description

La présente invention a pour objet un procédé de détection et/ou de dosage d'une substance biologique selon une méthode d'affinité, ainsi qu'un coffret ou trousse de détection ou de dosage permettant la mise en oeuvre de ce procédé.

On sait que l'un des procédés les plus utilisés pour déterminer la présence ou doser, selon une méthode immunologique, les substances présentes dans un échantillon liquide, est la méthode dite "sandwich". Un premier anticorps, immobilisé sur un support solide, et reconnaissant un site antigénique (épitope) de la substance examinée, est mis en contact avec une solution contenant ladite substance ou susceptible de la contenir. Un second anticorps, marqué avec un agent traceur, et reconnaissant un épitope différent de celui qui est reconnu par le premier anticorps, est ajouté. On constate que lorsque les anticorps sont présents en quantité suffisante, les additions de la substance biologique et du deuxième anticorps peuvent être faites simultanément (méthode dite "simultanée"). Si la substance biologique recherchée est présente dans l'échantillon liquide étudié, on observe l'immobilisation du deuxième anticorps marqué grâce à la formation du complexe anticorps 1-substance biologique-anticorps 2.

Mais, lorsque la substance biologique (que l'on peut considérer ici comme un antigène) est présente en quantités importantes, l'excès d'antigènes peut conduire à des situations où l'antigène se fixe d'une part sur l'anticorps 1 immobilisé, et d'autre part sur l'anticorps 2 marqué, en solution, de sorte que seule une faible proportion de l'anticorps 2 se trouve fixée sur le support. En fin de réaction, la phase solide est revêtue essentiellement de l'antigène seul tandis que l'anticorps 2 portant l'agent traceur est présent en solution sous la forme d'un complexe avec l'antigène. Ainsi, le signal détecté tend vers zéro pour des concentrations très élevées en antigènes.

Ce phénomène, appelé effet de zone (en anglais : hook effect), est bien connu en immuno-chimie ; voir par exemple D. Rodbard et al., Immunochemistry, Vol. 15, pp 77-82 (1978) et K.L. Hoffman et al., Clinical Chemistry, 30 (9), pp 1499-1501 (1984).

Pour éliminer cet effet de zone, on a préconisé l'augmentation de la concentration de l'un au moins des deux anticorps, ce qui peut entrainer des défauts de spécificité.

Une autre solution à ce problème est de mélanger l'anticorps marqué avec le même anticorps non marqué tout en augmentant la concentration globale du premier et du second anticorps. Cette méthode est efficace, mais elle présente l'inconvénient que seule une partie des complexes anticorps 1-antigène-anticorps 2 sont marqués, ce qui entraîne une réduction de la sensibilité du système de révélation.

On a maintenant découvert qu'il est possible d'éviter les inconvénients qui viennent d'être exposés, en utilisant comme second anticorps, un anticorps capable de reconnaître spécifiquement la liaison anticorps 1-antigène

Les demandes de brevets GB-2161165, GB-2171999 et WO-85/04422 décrivent l'utilisation, dans la détermination d'un petit antigène (de masse moléculaire inférieure à 5000) ou d'un haptène, d'un premier anticorps capable de reconnaître le déterminant antigénique (unique) de l'antigène ou de l'haptène, et d'un second anticorps capable de reconnaître spécifiquement la combinaison de l'antigène ou de l'haptène avec le premier anticorps. Ces demandes de brevets ne suggèrent pas l'utilisation d'un système d'anticorps analogues dans la détermination d'antigènes, de masse moléculaire plus élevée, possédant plusieurs épitopes, dans le but d'éliminer les risques de résultats erronés dûs à un effet de zone.

Le procédé de l'invention peut donc être utilisé, notamment selon la méthode simultanée, lorsque la concentration de l'antigène, dans l'échantillon examiné, est susceptible de varier dans des proportions importantes. On peut alors éliminer totalement ou fortement l'effet de zone observé à forte concentration d'antigène.

Le procédé de l'invention permet donc notamment de résoudre les problèmes posés par la baisse ou l'extinction du signal détecté pour de fortes concentrations d'antigène dans un dosage immunologique avec incubation simultanée de l'anticorps fixé et de l'anticorps marqué.

En outre, le procédé de l'invention peut être appliqué des systèmes autres que ceux basés sur l'affinité antigène-anticorps, par exemple à des sondes nucléiques, dont les déterminations peuvent également être faussées par un effet de zone.

La présente invention a donc pour objet un procédé de détection et/ou de dosage d'une substance biologique dans un liquide la contenant ou susceptible de la contenir, selon une méthode connue en soi, à l'aide de deux ligands différents, ladite substance biologique possédant au moins deux sites de liaison avec des ligands différents, et la concentration de ladite substance biologique dans ledit liquide étant susceptible de varier dans des proportions allant de 1 à 100 ou davantage, procédé dans lequel on utilise un premier ligand ayant une affinité pour un site de ladite substance biologique, et un second ligand, et dans lequel un des ligands est marqué avec un agent traceur, caractérisé par le fait qu'afin d'éviter un effet de zone, le second ligand est

2

un ligand ayant une affinité spécifique pour le complexe formé par le premier ligand fixé par affinité sur ladite substance biologique.

Les substances biologiques auxquelles s'applique le procédé de l'invention ont des masses moléculaires supérieures à 5000, et généralement supérieures à 10.000 : c'est le cas en pratique pour les acides nucléiques (ADN ou ARN) et aussi pour les protéines possédant plusieurs épitopes différents. Le plus souvent, les substances biologiques auxquelles s'applique le procédé de l'invention ont une masse moléculaire pouvant aller de 20.000 à 500.000 environ.

Dans un mode de réalisation particulier, la substance biologique est un acide nucléique, le premier ligand est une première sonde nucléique (qui peut être fixée sur un support solide) dont une partie est capable de s'hybrider avec une première séquence dudit acide nucléique tandis que l'autre partie de la première sonde ne s'hybride pas avec la substance biologique, et le second ligand est une deuxième sonde nucléique (marquée avec un agent traceur) dont une partie est capable de s'hybrider avec une seconde séquence dudit acide nucléique seulement si l'autre partie de ladite deuxième sonde est capable de s'hybrider avec ladite autre parce de la première sonde, étant entendu que lesdites première et seconde séquences de la substance biologique sont suffisamment voisines pour permettre l'hybridation effective desdites autres parties. L'agent traceur est par exemple un agent radioactif ou enzymatique, ou un antigène capable d'être reconnu par un anticorps conjugué lui-même avec une enzyme ou un composé fluorescent ou luminescent.

Dans un autre mode de réalisation particulier de l'invention de ce procédé, les premier et second ligands sont des anticorps, premier anticorps reconnaissant un site antigénique caractéristique de la substance biologique et le second anticorps reconnaissant spécifiquement le complexe immun formé par le premier anticorps fixé sur la substance biologique. Dans ce cas, la substance biologique est notamment une protéine. Parmi les protéines dont la concentration est susceptible de varier fortement dans les échantillons à analyser, on citera, à titre d'exemples non limitatifs, l'hormone chorionogonadotrophine (hCG), la FSH (follicle stimulating hormone), la LH (luteinizing hormone), la TSh (thyroid stimulating hormone), l'alpha-foeto protéine, la protéine C réactive, les IgE, la ferritine, et divers antigènes de surface de bactéries, de virus ou de parasites, par exemple l'antigène P30 de toxoplasma gondii ou la glycoprotéine Gp41 de HIV1.

La présente invention a donc notamment pour objet un procédé de détection et/ou de dosage d'une substance biologique dans un échantillon liquide la contenant ou susceptible de la contenir, dans lequel on utilise un premier anticorps dirigé contre ladite substance biologique, et un second anticorps, caractérisé par le fait que le deuxième anticorps est un anticorps dirigé contre le complexe immun formé par le premier anticorps et ladite substance biologique.

L'un des deux anticorps peut être marqué par un agent traceur, et l'autre anticorps peut être fixé sur une phase solide.

Selon un mode d'exécution particulier, l'un au moins des deux anticorps est un anticorps monoclonal. De préférence les deux anticorps sont des anticorps monoclonaux.

Il est également possible d'ajouter le second anticorps avec un troisième anticorps marqué capable de reconnaître un épitope, de la substance biologique, différent de l'épitope reconnu par le premier anticorps. La détection se trouve ainsi améliorée, notamment pour les faibles concentrations de la substance biologique.

Selon un mode d'exécution particulier, lorsque le premier anticorps est fixé sur un support solide et le second anticorps est marqué avec un agent traceur, on met en contact l'échantillon liquide avec ledit premier anticorps et avec ledit second anticorps, et après des temps d'incubation convenables, on sépare la phase liquide du support solide et on détermine la présence et/ou la quantité de la substance biologique on ledit échantillon liquide par détermination de la présence et/ou de la quantité d'agent traceur fixé sur le support solide. Ce procédé peut notamment être mis en oeuvre selon la méthode simultanée, c'est-à-dire que l'on met en contact l'échantillon liquide à la fois avec les premier et deuxième anticorps simultanément, sans incubation intermédiaire, de façon à n'effectuer qu'une seule incubation.

Selon un autre mode d'exécution, le procédé de détection ou de dosage de l'invention peut être mis en oeuvre en solution, c'est-à-dire sans phase solide, en utilisant le fait que, dans le complexe anticorps 1-antigène-anticorps 2, les deux anticorps sont très proches l'un de l'autre, puisque le second anticorps reconnaît la zone de liaison entre l'anticorps 1 et l'antigène.

Ce mode de réalisation particulier par voie liquide, c'est-à-dire sans anticorps fixé sur une phase solide, est caractérisé par le fait que les deux anticorps sont marqués chacun par un agent traceur, et que les agents traceurs des deux anticorps sont interactifs. Par exemple, l'un des agents traceurs est un composé fluorescent et l'autre agent traceur est un composé capable d'éteindre la fluorescence. Ou bien l'un des anticorps est marqué par une enzyme qui catalyse une réaction impliquant une substance liée à l'autre anticorps. Ou bien encore, les deux marqueurs constituent ensemble un système multi-enzymatique capable de produire un signal lumineux.

De tels agents traceurs interactifs sont connus ; voir par exemple "Immunoassays in the Clinical Laboratory", Nakumura, Dito, Tucker Editeurs, Alain R. Liss Inc. New York, 1978, pages 211 - 226.

Les premiers anticorps utilisables pour la mise en oeuvre du procédé de la présente invention sont préparés selon les méthodes usuelles, par immunisation d'animaux vertébrés avec la substance biologique considérée. Les anticorps obtenus peuvent être purifiés par exemple par chromatographie d'affinité. De préférence, on transforme les cellules productrices d'anticorps des animaux immunisés en hybridomes, selon les méthodes connues, puis on clone les cellules hybrides produisant les anticorps intéressants.

Pour préparer le second anticorps, on immunise selon les techniques habituelles des animaux avec le complexe substance biologique-premier anticorps (le premier anti corps étant de préférence monoclonal). On a constaté que les animaux ainsi immunisés produisent une certaine proportion d'anticorps qui reconnaissent le complexe utilisé l'immunisation, mais qui ne reconnaissent de façon appréciable ni la substance biologique seule, ni le premier anticorps seul. De préférence, le second anticorps (anti-complexe) est un anticorps monoclonal obtenu par exemple selon la techniques des hybridomes.

Le marquage des anticorps avec un agent traceur (radioactif, fluorescent, enzymatique ou autre) est effectué selon les techniques connues. De même, la révélation du marqueur est effectuée de façon connue.

La fixation des anticorps sur un support solide est également connue. Les supports solides sont par exemple des plaques de microtitration, des tubes, des billes ou des barrettes de polystyrène, des microbilles de latex, etc... La fixation sur le support est faite par adsorption, par covalence, ou par l'intermédiaire d'un anticorps (lui-même fixé directement sur le support) reconnaissant un déterminant antigénique isotypique du premier anticorps. L'anticorps intermédiaire provient bien entendu d'une autre espèce animale que celle qui a servi à obtenir le premier anticorps.

L'invention a également pour objet un coffret ou trousse de détection, selon le procédé décrit précédemment, d'une substance biologique, caractérisé par le fait qu'il contient, outre des tampons et éventuellement des supports solides appropriés, au moins lesdits premier et second ligands tels que définis précédemment. Le coffret ou trousse de détection peut contenir en outre un agent de révélation de la présence de l'agent traceur.

Avec le procédé de l'invention, l'effet de zone aux fortes concentrations est suffisamment faible pour ne pas donner des résultats faussement négatifs. Ce faible effet de zone est susceptible de limiter les possibilités de quantification rigoureuse aux fortes concentrations d'antigène. Mais le résultat trouvé étant dans ce cas voisin du signal maximum, il suffit alors de diluer l'antigène jusqu'à des concentrations (qui peuvent être déterminées à l'avance) où le signal est facilement interprétable, pour pouvoir ensuite effectuer, toujours selon le procédé de l'invention, un dosage correct.

Les exemples suivants illustrent l'invention.

Exemple 1: Obtention d'anticorps et essais de détection de l'hCG.

Introduction: l'hormone hCG comporte deux sous-unités, alpha et bêta. Seule la sous-unité bêta porte une spécificité antigénique. La sous-unité alpha est commune à plusieurs hormones (FSH, LH, TSH, ...).

On sait que la concentration de cette hormone s'élève rapidement après la fécondation, ce qui constitue un indicateur sensible d'une grossesse débutante.

Une des difficultés du dosage de cette hormone, par les méthodes immunochimiques, réside donc dans la très grande gamme de concentrations sérique ou urinaire qu'il est possible de rencontrer. Le risque qu'un effet de zone vienne perturber la détermination de cette hormone empêche donc, en pratique, l'utilisation de la méthode classique "sandwich" simultanée. Cet effet n'est pas à redouter lorsqu'on utilise le procédé de l'invention.

Stade 1 : Obtention d'anticorps monoclonaux dirigés contre l'hCG.

L'antigène utilisé est l'hCG commercialisé par DIOSYNTH (Pays Bas), référence 8856 01, contenant 3020 U/mg de poids sec. La pureté de la préparation a été contrôlée par électrophorèse en gel d'acrylamide.

On rappelle qu'une unité d'hCG correspond à 3 000 µg d'hCG.

Immunisation : On immunise des souris Balb/c par injection par voie intrapéritonéale avec 30 U d'hCG, par injection et par souris. La première injection est réalisé avec de l'adjuvant complet de Freund, les autres injections à 4-8 semaines d'intervalle avec de l'adjuvant incomplet. Un dernier rappel est effectué 3 jours avant la fusion, avec 30 U d'HcC en tampon physiologique, par voie intraveineuse. Après ce rappel, on prélève la rate des souris immunisées et on recueille les splénocytes.

On procède alors à une opération de fusion des cellules spléniques avec des cellules de la lignée myélomateuse SP2/O-Ag14 (Nature, 276, 269-270, 1978).

Après sélection des cellules hybrides viables sécrètant des anticorps anti-hCG (repérés par test ELISA) et clonage selon les méthodes usuelles, on a obtenu des lignées de cellules hybrides sécrètant des anticorps monoclonaux reconnaissant l'hCC.

Ces anticorps reconnaissent soit la chaîne alpha, soit la chaîne bêta, soit les chaînes alpha et bêta de l'hCC.

Les anticorps monoclonaux ont été produits à la fin du stade 1 sur ascites de souris et purifiés par chromatographie sur échangeur d'ions (DEAE Trisacryl, commercialisé par IBF réf. 250-771), avec élution par une solution aqueuse d'un gradient de phosphate de sodium. La concentration en phosphate de sodium du tampon d'élution est voisine de 15 mM.

Stade 2 : obtention d'anticorps monoclonaux dirigés contre la zone de liaison d'un anticorps monoclonal anti-chaîne bêta de l'hCG.

On prépare un complexe immun avec l'un des anticorps monoclonaux anti-chaîne bêta de l'hCG, obtenu au stade précédent (appelé ci-après anticorps anti-bêta) et l'hCG, par mélange équimolaire.

La solution de complexe immun ainsi obtenue est utilisée comme immunogène pour immuniser des souris Balb C, selon la méthode décrite précédemment. On prépare des cellules hybrides comme précédemment et on sélectionne les cellules sécrètant des anticorps qui reconnaissent en ELISA le complexe immun utilisé pour l'immunisation.

On sélectionne finalement les clones produisant les anticorps spécifiques du complexe immun, qui ne reconnaissent pas l'hCG (c'est-à-dire qu'ils ne reconnaissent ni la chaîne alpha ni la chaîne bêta) et qui ne reconnaissent pas non plus l'anticorps monoclonal anti-bêta ayant servi à préparer le complexe immun utilisé comme agent d'immunisation.

On a retenu pour la suite des expériences , trois anticorps monoclonaux spécifiques ayant une bonne affinité spécifique pour le complexe immun.

Ces anticorps sont appelés ci-après anti-complexe 1, anti-complexe 2 et anti-complexe 3, respectivement.

Ces anticorps sont purifiés comme au stade 1. Ils sont ensuite marqués soit par la phosphatase alcaline, soit par la peroxydase de raifort, selon la méthode décrite par NAKANE (réf. Journal of Histoch and Cytochem, 1974, Vol 22, N° 12, pages 1084 - 1091).

Stade 3 : Essai de détection de l'hCG.

L'anticorps anti-bêta est fixé par adsorption sur une plaque de microtitration.

On utilise comme deuxième anticorps l'anticorps anti-complexe 1 conjugué à la phosphatase alcaline, sous la forme d'une solution aqueuse contenant du phosphate de sodium et du sérum de cheval.

Les essais sont effectués sur des solutions à diverses concentrations d'hCG (de 0 à 500 µg/ml).

Les réactifs sont incubés simultanément pendant une heure. Après lavage de la plaque, le substrat de la phosphatase alcaline, c'est-à-dire le PNPP (para nitrophényl phosphate) a été ajouté à raison de 0,2 mg par puits, et au bout d'une heure, les densités optiques ont été mesurées.

Les résultats sont résumés dans le tableau 1 suivant :

### TABLEAU 1

| Concentration en hCG en µg/ml | 0 | 10 | 50 | 100 | 250 | 500 |
|---|---|---|---|---|---|---|
| Densité optique à 405 nm | 0,000 | 0,094 | 0,154 | 0,241 | 0,455 | 0,617 |

Les résultats du tableau 1 montrent qu'il n'y a pas d'effet de zone pour des concentration en hCG comprises entre 0 et 500 µg/ml.

On a refait la même expérience, mais en remplaçant l'anticorps anti-bêta par un anticorps monoclonal anti-alpha obtenu au stade 1.

Les résultats obtenus sont résumés dans le tableau 2 suivant :

TABLEAU 2

| Concentration en hCG en µg/ml | 0 | 10 | 50 | 100 | 250 | 500 |
|---|---|---|---|---|---|---|
| Densité optique à 405 nm | 0,044 | 0,032 | 0,024 | 0,024 | 0,026 | 0,029 |

Les résultats du tableau 2 montrent que l'anticorps anti-complexe 1 ne reconnaît pas la zone de liaison de l'hCG avec l'anticorps anti-alpha.

Exemple 2 : On a procédé à des expériences similaires à celles décrites à l'exemple 1, stade 3 en utilisant comme anticorps immobilisé l'anti-bêta et comme second anticorps l'anti-complexe 1, marqué à la phosphatase alcaline.

On a comparé les résultats avec ceux obtenus dans un test "sandwich" classique, avec comme second anticorps, un anticorps monoclonal anti-alpha obtenu à l'exemple 1, stade 1.

Les densités optiques obtenues dans les deux cas sont mentionnées dans le tableau 3 suivant :

TABLEAU 3

| gamme d'hCG en µg/ml | 0 | 0,01 | 0,1 | 1 | 10 | 100 | 1000 |
|---|---|---|---|---|---|---|---|
| anti-complexe 1 | 0,000 | 0,060 | 0,263 | 1,391 | 2,212 | 2,202 | 2,055 |
| anti-alpha | 0,012 | 0,041 | 1,291 | 2,574 | 2,524 | 1,295 | 0,261 |

Les résultats du tableau 3 montrent que l'effet de diminution du signal à forte concentration en antigènes est négligeable pour l'anticorps anti-complexe 1, alors qu'il est très marqué pour l'anticorps anti-alpha.

Exemple 3 : Des essais analogues ont été réalisés avec des anticorps marqués à la peroxydase selon le protocole d'incubation simultanée des réactifs suivants :

- Anticorps immobilisé : anti-bêta
- Antigène : hCG
- Deuxième anticorps, marqué à la peroxydase : anti-complexe 1 ou anti-alpha.

Après incubation pendant une heure à 37°C, on lave la plaque de microtitration puis on ajoute le substrat OPD (ortho phénylène diamine) et on laisse incuber pendant 5 minutes. On procède ensuite à la lecture des densités optiques à la longueur d'ondes 492 nm.

Les résultats sont résumés dans le tableau 4 suivant :

TABLEAU 4

| hCG en µg/ml | 0 | 0,01 | 0,1 | 1 | 10 | 100 | 1000 |
|---|---|---|---|---|---|---|---|
| anti-complexe 1/ peroxydase | 0,000 | 0,145 | 0,380 | 1,334 | 1,149 | 1,126 | 0,962 |
| anti-alpha/ peroxydase | 0,000 | 0,825 | 1,338 | 1,405 | 0,753 | 0,082 | 0,000 |

Les résultats du tableau 4 montrent que la décroissance de densité optique à forte concentration est très faible avec l'anti-complexe 1 alors que la densité optique devient nulle à forte concentration (1 mg/ml) pour le test "sandwich" utilisant un anticorps anti-alpha.

Exemple 4 : Les essais sont réalisés avec les trois anticorps anti-complexe obtenus à l'exemple 1, stade 3, conjugués à la peroxydase.

Le protocole d'incubation est le suivant :
- anticorps anti-bêta fixé sur la plaque de microtitration par adsorption.
- incubation simultanée pendant une heure à 37°C de l'antigène hCG et de l'anticorps anti-complexe marqué à la peroxydase, ou de l'anticorps anti-alpha marqué à la peroxydase, ou d'un mélange 75:25 (en masse) d'anti-complexe et d'anti-alpha.
- Après lavage de la plaque, on ajoute le substrat (OPD).
- Après 15 minutes d'incubation et blocage de la réaction par addition d'une solution 1N d'acide sulfurique, on procède à la lecture des densités optiques à 492 nm.

La gamme d'étalonnage d'hCG s'étend de 0 à 200.000 U/l. Elle est préparée par dilutions à partir d'un étalon standard à 10.000U/ml.

Les résultats sont représentés sur la figure unique annexée, sous la forme d'un graphique, avec en abscisses le logarithme de la concentration en hCG (en unités par litre) et en ordonnées la densité optique (DO).

On voit sur la figure que dans le test "sandwich" utilisant l'anticorps anti-alpha, la densité optique décroît fortement pour les concentrations élevées en antigène.

Avec les anticorps anti-complexe 1 et anti-complexe 2, de même qu'avec les mélanges anti-complexe 1/anti-alpha et anti-complexe 2/ anti-alpha, il n'y a pas de baisse du signal à forte concentration en hCG.

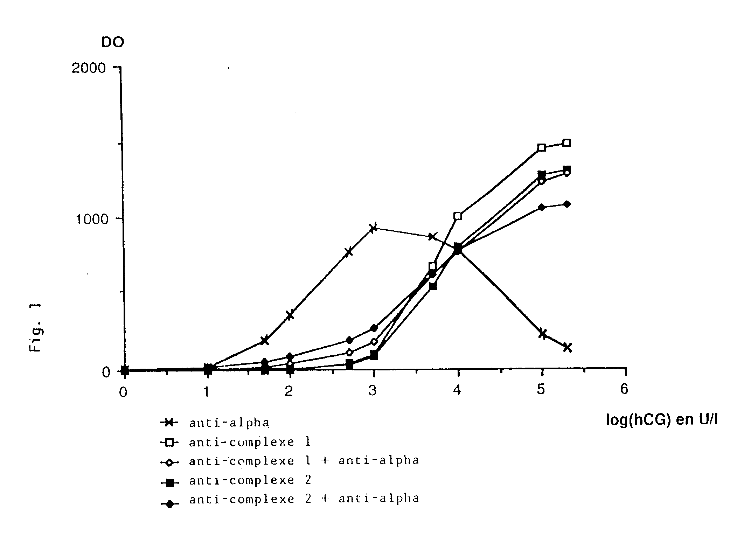

On voit également sur la figure 1 que le mélange d'un anticorps anti-complexe et de l'anti-alpha permet d'améliorer la sensibilité à faible concentration d'antigène.

Exemple 5 : Des dosages réels ont été réalisés sur 60 sérums dont les valeurs étaient comprises entre 0 et 5.000 U/l On a comparé les résultats obtenus avec les réactifs utilisés dans l'exemple 4 et un réactif commercial vendu par la Société HYBRITECH (coffret Tandem hCG réf. 41 784 70).

Le réactif commercial contient des anticorps monoclonaux anti-alpha- et anti-bêta-hCG. Avec les anticorps de ce réactif commercial, on réalise donc un test "sandwich" classique.

Les droites de corrélation des 60 sérums sont portés dans Le tableau 5, avec y représentant les résultats obtenus avec l'anticorps anti-bêta fixé sur la phase solide et les conjugués anti-alpha, anti-complexe, ou les mélanges anti-alpha/anti-complexe, et x représentant les résultats obtenus avec le réactif commercial.

Les proportions des mélanges d'anticorps sont les mêmes qu'à l'exemple 4.

Les résultats sont résumés dans le tableau 5 suivant, dans lequel on désigne par "conjugué" l'anticorps marqué à la peroxydase.

TABLEAU 5

| Conjugué | Droite de régression | Coefficient de corrélation |
|---|---|---|
| **Anti-alpha** | | |
| Conjugué anti-alpha | y = 0,165x + 290 | r = 0,30 |
| **Anti-complexe** | | |
| Conjugué anti-complexe 2 | y = 0,855x + 26 | r = 0,95 |
| Conjugué anti-complexe 2 + conjugué anti-alpha | y = 1,091x + 24 | r = 0,98 |
| Conjugué anti-complexe 3 | y = 0,702x + 17 | r = 0,94 |
| Conjugué anti-complexe 3 + conjugué anti-alpha | y = 1,006x + 15 | r = 0,98 |

Les résultats indiqués dans le tableau 5 montrent que, du fait des teneurs élevées en hCG de certains sérums, le conjugué anti-alpha conduit à une mauvaise corrélation. L'utilisation de mélanges de conjugués anti-alpha et anti-complexe améliore la précision de la détermination et conduit à de très bonnes corrélations avec le réactif de référence.

Exemple 6: On a obtenu des anticorps (Ac) monoclonaux anti-IgE humaine selon le protocole expérimental décrit au stade 1 de l'exemple 1, puis on a préparé un complexe immun avec l'un des Ac monoclonaux anti IgE obtenu au stade précédent (appelé ci-après Ac anti-IgE 1) et l'IgE humaine par mélange équimoléculaire. La solution du complexe immun ainsi obtenue a été utilisée comme immunogène pour immuniser des souris Balb C selon les techniques conventionnelles. On a préparé des cellules hybrides selon les techniques conventionnelles et or a sélectionné les cellules secrétant des Ac qui reconnaissent en ELISA le complexe immun utilisé pour l'immunisation. Finalement, on a sélectionné les clones qui produisent des Ac spécifiques du complexe immun (c'est-à-dire qui ne reconnaissent ni l'IgE seule, ni l'Ac anti-IgE 1 seul). On a retenu un Ac monoclonal spécifique (ci-après appelé anti-complexe 1') ayant une bonne affinité spécifique pour le complexe immun. Cet Ac a été ensuite purifié comme décrit au stade 1 de l'exemple 1 et a été marqué soit par la peroxydase de raifort selon la méthode de Nakane, soit par un traceur radioactif($^{125}$I).

Comparaison de résultats obtenus entre une technique "sandwich" conventionelle et le procédé de l'invention

Technique "sandwich" classique

L'Ac anti IgE-1 est fixé par adsorption sur un tube en polystyrène. On utilise comme deuxième Ac un Ac anti IgE-2, reconnaissant un épitope opposé à celui reconnu par l'anti-IgE 1, l'Ac anti-IgE 2 ayant été au préalable marqué par un traceur radioactif ($^{125}$I). Les réactifs, IgE (50 µl) - Ac anti IgE 1 - Ac anti IgE 2 (200 µl), sont incubés pendant des heures sous agitation.

8

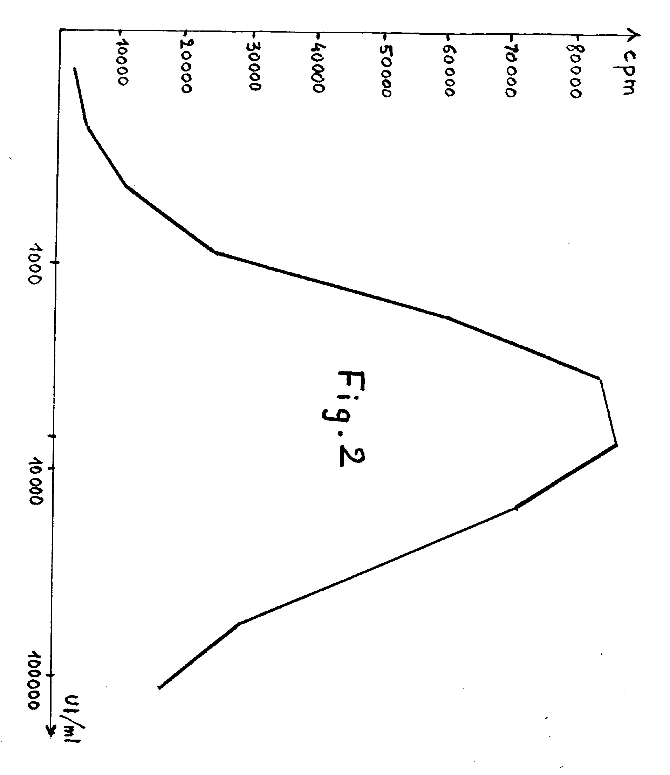

Des essais sont effectués en faisant varier la concentration d'IgE. Les résultats sont représentés sur la Figure 2 annexée, sur laquelle on a représenté en abscisses la concentration (UI/ml ; échelle logarithmique) et en ordonnées le nombre de coups par minute (cpm).

Le comptage obtenu se traduit par une courbe en "cloche" présentant un maximum de signal pour une concentration d'environ 7000 UI/ml d'IgE. Au-delà de cette valeur, le signal décroît lorsque la concentration en Ag augmente. L'effet de zone se traduit par un signal erroné par défaut à partir d'environ 50.000 UI/ml.

Procédé de l'invention

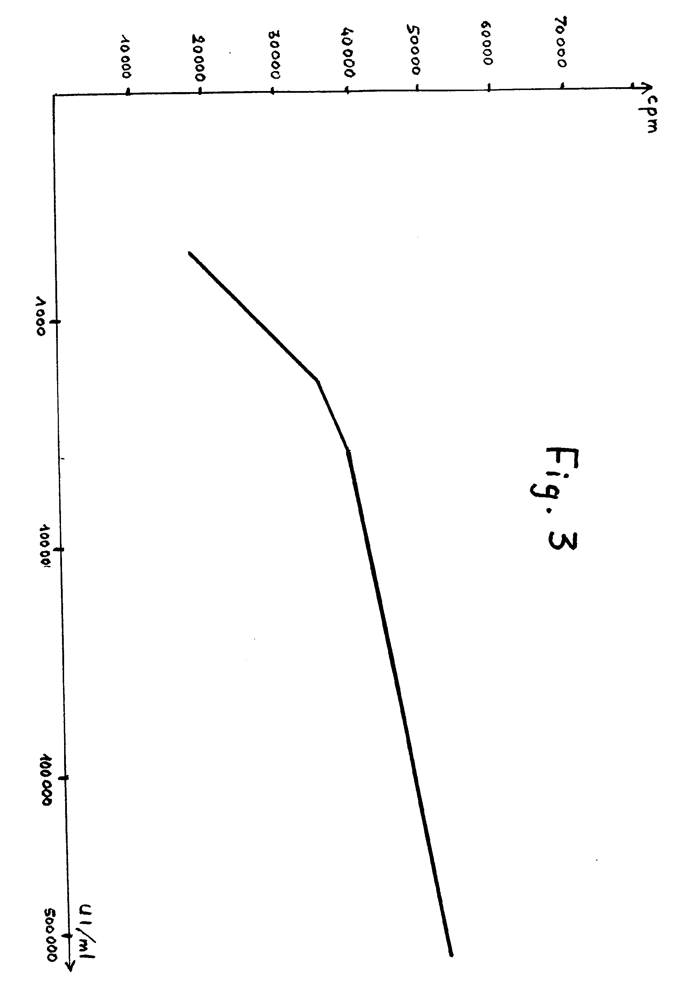

Dans les mêmes conditions, des essais ont été réalisés en utilisant comme Ac secondaire l'anti-complexe 1′ marqué à $^{125}$I. Comme cela ressort de la figure 3 annexée, même à des concentrations de 500.000 UI/ml, aucune baisse de signal n'est observée. Sur la figure 3, on a représenté en abscisses la concentration (échelle logarithmique) et en ordonnées les cpm.

Des dosages ont été réalisés sur 15 sérums dont les valeurs étaient comprises entre 0 et 1.000 UI/ml On a comparé les résultats obtenus avec un réactif disponible dans le commerce (BIOMERIEUX ref. 69200) et les réactifs de l'invention. Le réactif commercial contient des Ac monoclonaux anti IgE 1 et anti IgE2. Le réactif de l'invention contient des Ac monoclonaux anti IgE 1 et anti-complexe 1′. La corrélation obtenue suit la loi :

$$y = 0,96 \, x + 19$$

avec un coefficient de corrélation r = 0,98. La corrélation obtenue montre le bon accord entre les valeurs trouvées.

Exemple 7

Des essais analogues ont été effectués avec l'alpha-foeto protéine et trois Ac monoclonaux ont été obtenus, qui ont été testés selon les protocoles décrits précédemment. Les résultats sont analogues à ceux obtenus avec l'hCG et l'IgE.

## Revendications

1. Procédé de détection et/ou de dosage d'une substance biologique dans un liquide la contenant ou susceptible de la contenir, selon une méthode conventionnelle, à l'aide de deux ligands différents, ladite substance biologique possédant au moins deux sites de liaison avec des ligands différents, et la concentration de ladite substance biologique dans ledit liquide étant susceptible de varier dans des proportions allant de 1 à 100 ou davantage, procédé dans lequel on utilise un premier ligand ayant une affinité pour un site de ladite substance biologique, et un second ligand, et dans lequel un des ligands est marqué avec un agent traceur, caractérisé par le fait qu'afin d'éviter un effet de zone, le second ligand est un ligand ayant une affinité spécifique pour le complexe formé par le premier ligand fixé par affinité sur ladite substance biologique.

2. Procédé selon la revendication 1, caractérisé par le fait que ladite substance biologique a une masse moléculaire supérieure à 5000.

3. Procédé selon la revendication 1, caractérisé par le fait que ladite substance biologique a une masse moléculaire supérieure à 10.000.

4. Procédé selon la revendication 1, caractérisé par le fait que ladite substance biologique a une masse moléculaire pouvant aller d'environ 20.000 à 500.000.

5. Procédé selon l'une quelconque des revendications précédentes, caractérisé par le fait que lesdits premier et second ligands sont respectivement un premier et un second anticorps, et que le second anticorps reconnaît spécifiquement le complexe immun formé par le premier anticorps et ladite substance biologique.

6. Procédé selon la revendication 5, caractérisé par le fait qu'au moins un des deux anticorps est un anticorps monoclonal.

7. Procédé selon l'une quelconque des revendications 5 et 6, caractérisé par le fait que ladite substance biologique est une protéine.

8. Procédé selon l'une quelconque des revendications 5 à 7, caractérisé par le fait que ledit premier anticorps est fixé sur un support solide et ledit second anticorps est marqué avec un agent traceur.

9. Procédé selon la revendication 8, caractérisé par le fait que l'on met en contact ledit échantillon liquide avec ledit premier anticorps et avec ledit second anticorps, et qu'après des temps d'incubation convenables, on sépare la phase liquide du support solide et on évalue la présence et/ou la quantité de la substance biologique dans ledit échantillon liquide par détermination de la présence et/ou de la quantité d'agents traceur fixée sur le support solide.

10. Procédé selon la revendication 9, caractérisé par le fait que l'on met en contact ledit échantillon liquide à la fois avec les premier et deuxième anticorps simultanément, de façon à n'effectuer qu'une seule incubation.

11. Procédé selon l'une quelconque des revendications 5 à 10, caractérisé par le fait que ladite substance biologique est choisie parmi l'hCG, la FSH, la LH, la TSH, l'alpha-foeto protéine, la protéine C - réactive, les IgE, la ferritine et des antigènes de surface de bactéries, de virus ou de parasites.

12. Procédé selon la revendication 11, caractérisé par le fait que le premier anticorps est dirigé contre la chaîne bêta de l'hCG.

13. Procédé selon l'une quelconque des revendications 5 à 12, caractérisé par le fait que l'on utilise le second anticorps avec un troisième anticorps marqué capable de reconnaître un épitope de ladite substance biologique, autre que l'épitope reconnu par ledit premier anticorps.

14. Procédé selon l'une quelconque des revendications 1 à 4, caractérisé par le fait que la substance biologique est un acide nucléique, le premier ligand est une première sonde nucléique dont une partie est capable de s'hybrider avec une première séquence dudit acide nucléique tandis que l'autre partie de la première sonde ne s'hybride pas avec la substance biologique, et le second ligand est une deuxième sonde nucléique (marquée avec un agent traceur) dont une partie est capable de s'hybrider avec une seconde séquence dudit acide nucléique seulement si l'autre partie de ladite deuxième sonde est capable de s'hybrider avec ladite autre partie de la première sonde, étant entendu que lesdites première et seconde séquences de la substance biologique sont suffisamment voisines pour permettre l'hybridation effective desdites autres parties.

15. Coffret ou trousse de détection et/ou de dosage, selon le procédé de l'une quelconque des revendications précédentes, d'une substance biologique, caractérisé par le fait qu'il contient, outre des tampons et éventuellement des supports appropriés, au moins lesdits premier et second ligands.

16. Coffret ou trousse de détection selon la revendication 15, caractérisé par le fait qu'il comprend en outre un agent de révélation de la présence de l'agent traceur.

## Patentansprüche

1. Verfahren zum Nachweis und/oder der Dosierung einer biologischen Substanz in einer Flüssigkeit, die diese enthält oder geeignet ist, diese zu enthalten, gemäß einem üblichen Verfahren mit Hilfe zweier verschiedener Liganden, wobei die biologische Substanz mindestens zwei Bindungsstellen für die unterschiedlichen Liganden besitzt und die Konzentration der biologischen Substanz in der aufnahmefähigen Flüssigkeit in Anteilen von 1 bis 100 oder mehr variieren kann und wobei das Verfahren, in welchem man einen ersten Liganden mit einer Affinität gegen eine Bindungsstelle der biologischen Substanz und einen zweiten Liganden verwendet und in welchem einer dieser Liganden mit einem Tracer markiert ist, dadurch gekennzeichnet ist, daß zum Vermeiden eines Zoneneffektes (Hook effect) der zweite Ligand ein Ligand ist, der eine spezifische Affinität gegenüber dem aus dem ersten Liganden mit der biologischen Substanz gebildeten Komplex aufweist, an den er aufgrund seiner Affinität gebunden ist.

2. Verfahren gemäß Anspruch 1, dadurch gekennzeichnet, daß die biologische Substanz ein Molekulargewicht oberhalb von 5000 hat.

3. Verfahren gemäß Anspruch 1, dadurch gekennzeichnet, daß die biologische Substanz ein Molekulargewicht oberhalb von 10000 hat.

4. Verfahren gemäß Anspruch 1, dadurch gekennzeichnet, daß die biologische Substanz ein Molekulargewicht von etwa 20000 bis 500000 aufweist.

5. Verfahren gemäß einem der vorstehenden Ansprüche, dadurch gekennzeichnet, daß der erste und zweite Ligand jeweils ein erster bzw. zweiter Antikörper ist und daß der zweite Antikörper in spezifischer Weise den aus dem ersten Antikörper und der biologischen Substanz gebildeten Immunkomplex erkennt.

6. Verfahren gemäß Anspruch 5, dadurch gekennzeichnet, daß mindestens einer der beiden Antikörper ein monoklonaler Antikörper ist.

7. Verfahren gemäß einem der Ansprüche 5 und 6, dadurch gekennzeichnet, daß die biologische Substanz ein Protein ist.

8. Verfahren gemäß einem der Ansprüche 5 bis 7, dadurch gekennzeichnet, daß der erste Antikörper auf einem festen Träger fixiert ist und der zweite Antikörper mit einem Tracer markiert ist.

9. Verfahren gemäß Anspruch 8, dadurch gekennzeichnet, daß man eine flüssige Probe mit dem ersten und dem zweiten Antikörper in Kontakt bringt, nach einer angemessenen Inkubationszeit die flüssige Phase von dem festen Träger trennt und daß man das Vorhandensein und/oder die Menge der biologischen Substanz in der flüssigen Probe bestimmt, in dem man die Anwesenheit von und/oder die Menge an Tracer bestimmt, der auf dem Träger gebunden wurde.

10. Verfahren gemäß Anspruch 9, dadurch gekennzeichnet, daß man die flüssige Probe gleichzeitig mit dem ersten und dem zweiten Antikörper auf die Weise in Kontakt bringt, daß man nur eine einzige Inkubation durchführt.

11. Verfahren gemäß einem der Ansprüche 5 bis 10, dadurch gekennzeichnet, daß die biologische Substanz ausgewählt ist aus HCG, FSH, LH, TSH, α-Foetusprotein, einem reactivierten Protein C, den IgE, Ferritin und den Oberflächenantigenen von Bakterien, Viren oder Parasiten.

12. Verfahren gemäß Anspruch 11, dadurch gekennzeichnet, daß der erste Antikörper gegen die β-Kette des HCG gerichtet ist.

13. Verfahren gemäß einem der Ansprüche 5 bis 12, dadurch gekennzeichnet, daß man den zweiten Antikörper zusammen mit einem dritten markierten Antikörper verwendet, der in der Lage ist, einerseits ein Epitop der biologischen Substanz zu erkennen, und das sich von dem mittels des ersten Antikörpers erkannten Epitop unterscheidet.

14. Verfahren gemäß einem der Ansprüche 1 bis 4, dadurch gekennzeichnet, daß die biologische Substanz eine Nukleinsäure und der erste Ligand eine primäre Nukleinsonde ist, deren einer Teil in der Lage ist, mit einer ersten Sequenz der Nukleinsäure zu hybridisieren, während der andere Teil der ersten Sonde nicht mit der biologischen Substanz hybridisiert, und der zweite Ligand eine zweite Nukleinsonde (markiert mit einem Tracer) ist, deren einer Teil in der Lage ist, mit einer zweiten Sequenz auf der Nukleinsäure zu hybridisieren, einzig und allein dann, wenn der andere Teil der zweiten Sonde in der Lage ist, mit dem anderen Teil der ersten Sonde zu hybridisieren, wobei es selbstverständlich ist, daß die genannten ersten und zweiten Sequenzen auf der biologischen Substanz ausreichend benachbart sind, um eine effektive Hybridisierung der anderen Teile zu gestatten.

15. Ausstattung für den Nachweis und/oder die Dosierung einer biologischen Substanz gemäß einem der vorstehenden Ansprüche, dadurch gekennzeichnet, daß in ihr außer Puffern und eventuell geeigneten Trägern mindestens den ersten und zweiten Liganden enthalten ist.

16. Ausstattung für den Nachweis gemäß Anspruch 15, dadurch gekennzeichnet, daß in ihr darüber hinaus ein Mittel zur Bestimmung der Anwesenheit des Tracers enthalten ist.

EP 0 419 367 B1

## Claims

1. Process for the detection and/or assay of a biological substance in a liquid which contains it or which is liable to contain it, according to a standard method, with the aid of two different ligands, the said biological substance having at least two binding sites for different ligands, and the concentration of the said biological substance in the said liquid being capable of varying within proportions ranging from 1 to 100 or more, in which process a first ligand, having an affinity for a site of the said biological substance, and a second ligand, are used, and in which process one of the ligands is labelled with a tracer agent, characterized in that, in order to avoid a hook effect, the second ligand is a ligand which has a specific affinity for the complex formed by the first ligand which is bound by affinity to the said biological substance.

2. Process according to Claim 1, characterized in that the said biological substance has a molecular mass greater than 5,000.

3. Process according to Claim 1, characterized in that the said biological substance has a molecular mass greater than 10,000.

4. Process according to Claim 1, characterized in that the said biological substance has a molecular mass which can range from approximately 20,000 to 500,000.

5. Process according to any one of the preceding claims, characterized in that the said first and second ligands are, respectively, a first and a second antibody, and in that the second antibody specifically recognizes the immune complex formed by the first antibody and the said biological substance.

6. Process according to Claim 5, characterized in that at least one of the two antibodies is a monoclonal antibody.

7. Process according to either of Claims 5 and 6, characterized in that the said biological substance is a protein.

8. Process according to any one of Claims 5 to 7, characterized in that the said first antibody is bound on a solid support and the said second antibody is labelled with a tracer agent.

9. Process according to Claim 8, characterized in that the said liquid sample is placed in contact with the said first antibody and with the said second antibody, and in that, after suitable incubation times, the liquid phase is separated from the solid support and the presence and/or the quantity of the biological substance in the said liquid sample is evaluated by determination of the presence and/or the quantity of tracer agents which are bound to the solid support.

10. Process according to Claim 9, characterized in that the said liquid sample is placed in contact with both the first and the second antibodies simultaneously, so that only a single incubation is carried out.

11. Process according to any one of Claims 5 to 10, characterized in that the said biological substance is chosen from hCG, FSH, LH, TSH, alpha-foetoprotein, C-reactive protein, IgE, ferritin and surface antigens of bacteria, viruses or parasites.

12. Process according to Claim 11, characterized in that the first antibody is directed towards the beta chain of hCG.

13. Process according to any one of Claims 5 to 12, characterized in that the second antibody is used with a labelled third antibody which is capable of recognizing an epitope of the said biological substance, other than the epitope recognized by the said first antibody.

14. Process according to any one of Claims 1 to 4, characterized in that the biological substance is a nucleic acid, the first ligand is a first nucleic acid probe, one portion of which is capable of hybridizing with a first sequence of the said nucleic acid while the other portion of the first probe does not hybridize with the biological substance, and the second ligand is a second nucleic acid probe (labelled with a tracer agent), one portion of which is capable of hybridizing with a second sequence of the said nucleic acid only if the other portion of the said second probe is capable of hybridizing with the said other portion of the first probe, it being understood that the said first and second sequences of the biological substance are suf-

12

ficiently close together to permit effective hybridization of the said other portions.

15. Kit or outfit for the detection and/or assay, according to the process of any one of the preceding claims, of a biological substance, characterized in that it contains, in addition to buffers and, where appropriate, suitable supports, at least the said first and second ligands.

16. Detection kit or outfit according to Claim 15, characterized in that it additionally comprises an agent for visualizing the presence of the tracer agent.

Fig. 1

Fig. 2

Fig. 3

U I/ml

cpm

1000    10000    100000    500000

10000    20000    30000    40000    50000    60000    70000

EP 0 419 367 B1